# EUROPEAN PATENT APPLICATION

(11) **EP 4 566 483 A1**
(43) Date of publication of application: **11.06.2025**
(21) Application number: 23215044.1
(22) Date of filing: 07.12.2023
(51) Int. Cl.: A46B 15/00, A61B 5/00, G06T 7/00

(54) **SYSTEM FOR DETECTING DENTAL PLAQUE**

(71) Applicant: Koninklijke Philips N.V., 5656 AG Eindhoven (NL)
(72) Inventor: GOTTENBOS, Bart, Eindhoven (NL); CIUHU, Calina, Eindhoven (NL); KOOIJMAN, Gerben, Eindhoven (NL)
(74) Representative: Philips Intellectual Property & Standards

(57) **Abstract**

A system and method are provided for detecting dental plaque. Intra-oral images are generated comprising light induced fluorescence images. Shape and/or intensity distribution features of fluorescent regions in the generated images are identified so that regions of tartar and regions of plaque can be discriminated.

## Description

### FIELD OF THE INVENTION

This invention relates to systems for detecting dental plaque, in particular based on image analysis. It for example relates to a detection system that may be integrated with an electric toothbrush.

### BACKGROUND OF THE INVENTION

Oral hygiene is an important element in maintaining oral health. In addition to routine dentist visits, consumers express the need to monitor more frequently their oral health and receive guidance and reassurance on how well the teeth cleaning is performed.

It is known to use camera-based sensing to monitor oral hygiene. Image sensing has been proposed for detection of plaque, tartar, stains, gum health and caries.

One approach uses blue or violet light to illuminate the teeth and detect red fluorescent spots, a known technology called quantified light fluorescence (QLF). Red fluorescent spots can be older plaque, meaning these spots have not been cleaned for a few days, but also tartar and stains can provide strong red fluorescent signals. This information can then be translated into the toothbrush handle to guide people better during brushing to clean away the old plaque, or to even change the brush parameters to better address old plaque.

However, a high fluorescence signal during QLF imaging might equally indicate plaque or tartar. In providing feedback and guidance to the user, discriminating between the two is highly desired, as plaque is actionable (i.e., can be removed with brushing), whereas tartar and staining is not. Tartar and stains may be addressed through dental professional treatment.

US11471034 discloses the analysis of a fluorescence signal and uses an overall intensity to discriminate between plaque and tartar. However, this is not a very reliable method to distinguish between plaque and tartar, as old plaque also can have very bright red autofluorescence.

### SUMMARY OF THE INVENTION

The invention is defined by the claims.

According to examples in accordance with an aspect of the invention, there is provided a system for detecting dental plaque, comprising:
a camera system for generating intra-oral images comprising a light source and a detector for generating light induced fluorescence images;
a processor for processing the generated images, configured to:
   detect shape and/or intensity distribution features of fluorescent regions in the generated images; and
   discriminate between regions of tartar and regions of plaque from the shape and/or intensity distribution features.

It is known to use image sensing (for example with a simple QLF camera) for detection of plaque, tartar or other features, but without any discrimination technologies.

The invention is based on the recognition that the appearance of red fluorescent tartar is slightly different from red fluorescent plaque. Plaque has a spotty appearance whereas tartar is more homogeneous in intensity and also has straighter edges. The invention makes use of algorithms running on a processor to detect one or more of these differences.

The system of the invention thus enables users to have an improved experience as the system can provide a plaque indication when plaque is detected, but not when there is a very high likelihood that the detected red fluorescence relates to tartar. This avoids the system advising the user that there are plaque areas, but the user will not be able to brush them away because they are tartar.

The image analysis may additionally detect a stained spot, carious lesion or crack. Thus, while the system of the invention is of primary interest for discriminating between plaque and tartar it also can be extended for detection of a stained spot, carious lesion or crack.

The light source of the camera for example is a blue or violet light source. As is well known, blue light may be used to illuminate the teeth for QLF imaging. This causes the teeth to fluoresce in green (autofluorescence), whereas red fluorescence is caused as a result of the metabolic process of specific bacterial strains.

The shape and/or intensity distribution features for example comprise an assessment of spatial homogeneity of fluorescence within the fluorescent regions. Alternatively, or additionally, the shape and/or intensity distribution features may comprise an assessment of edge sharpness. These are characteristics that have been found to be able to discriminate between plaque and tartar. Thus, "shape and/or intensity distribution features" are intended to relate to the external shape of a general area or the internal pattern characteristics such as intensity variations over the general detection area within which the fluorescence signal is present.

In one set of examples, the processor is configured to run a non-intelligent, i.e., non-trained, algorithm to analyze the fluorescent regions. In this way, a low-cost algorithm with low computational resource requirements may be used. The algorithm may use a conventional image processing-based approach (for example comprising intensity-based clustering of pixels and use of a decision tree). This is particularly relevant when the plaque detection is run real-time off-cloud (for example embedded in an oral care device, or on a local remote device with which the oral care device communicates, such as a mobile phone running an app).

For example, the processor may be configured to determine a circumference to area ratio to determine an edge sharpness. This edge sharpness may be for a general area within which the fluorescent signal exists (with a level above a threshold).

The processor may be configured to perform red and/or green channel thresholding, generate histogram data, and analyze the histogram data to discriminate between regions of tartar and regions of plaque. Again, this histogram may be for a general area within which the fluorescent signal exists (with a level above a threshold).

By red "and" green channel thresholding is for example meant thresholding a combination (e.g., subtraction or division) of the red and green channels.

The processor may be configured to determine a spatial homogeneity of fluorescence by:
performing measures of local entropy of the fluorescent regions; or
performing spatial frequency analysis of the fluorescent regions.

Values of local entropy are higher in spotty areas, indicating presence of plaque rather than regular tartar deposition. Similarly, there are greater high spatial frequency components in spotty areas than in homogeneous areas.

These are example of texture analysis. Any suitable texture analysis may be used that can provide a measure of uniformity of the intensity values over the area of interest.

In another set of examples, the processor is configured to run a trained AI algorithm to analyze the fluorescent regions. This may be trained using images annotated by a dental practitioner as including tartar or plaque (or none).

The system may further comprise an output system configured to provide a notification when plaque is determined to be present. This notification is for example used to indicate that a particular area should be brushed more thoroughly to remove the plaque.

The output system may be further configured to provide brushing guidance to a user. This will assist the user in developing a brushing technique which reduces plaque or prevents its formation, by spending more time at plaque vulnerable areas.

The invention also provides an oral care device comprising:
an oral care element; and
the detection system as defined above.

The detection system may in this way be integrated with an oral care device. Thus, in real time, the device can alert a user to the presence of plaque so that remedial action can be taken. The oral care device is for example a powered toothbrush, wherein the oral care element comprises a driven toothbrush head.

The processor of the powered toothbrush is for example configured to adapt operating parameters of the powered toothbrush in response to the detection of regions of plaque.

The invention also provides a method for detecting dental plaque, comprising:
receiving intra-oral images comprising light induced fluorescence images;
detecting shape and/or intensity distribution features of fluorescent regions in the received images; and
discriminating between regions of tartar and regions of plaque from the shape and/or intensity distribution features.

The shape and/or intensity distribution features for example comprise an assessment of spatial homogeneity of fluorescence within the fluorescent regions and/or an assessment of edge sharpness.

The invention also provides a computer program comprising computer program code which is adapted, when said program is run on a processor, to perform the method as defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a detection system integrated into a power toothbrush;
Fig. 2 shows differences between red fluorescent images of plaque and tartar;
Fig. 3 shows differences between histogram data for plaque and tartar;
Fig. 4 shows differences in edge sharpness for plaque and tartar; and
Fig. 5 shows a method of detecting plaque.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method detecting dental plaque. Intra-oral images are generated comprising light induced fluorescence images. Shape and/or intensity distribution features of fluorescent regions in the generated images are identified so that regions of tartar and regions of plaque can be discriminated.

The invention may be a standalone system for detecting plaque, but more preferably it forms part of an oral care device such as an electric power toothbrush.

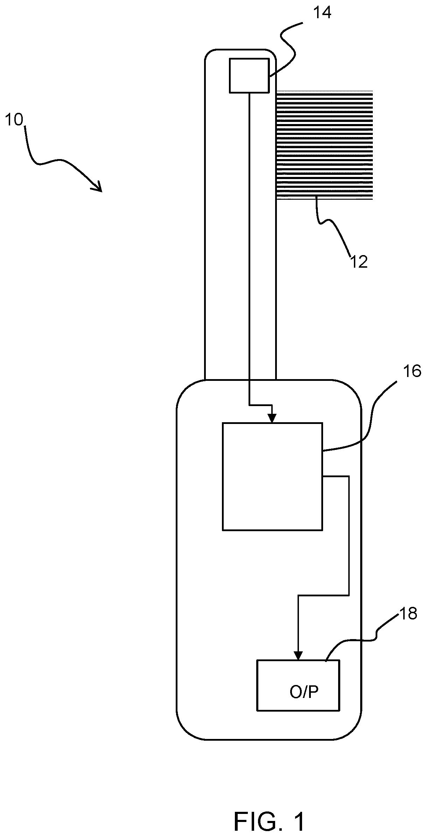

Fig. 1 shows an electric toothbrush 10 having a brush head 12 (which more generally is an oral care element) and a detection system. The detection system is integrated with the toothbrush. The toothbrush is able to alert a user to the presence of plaque so that remedial action can be taken. The detection system comprises a camera system 14 for generating intra-oral images and a processor 16 for processing the generated images. The camera system 14 comprises an illumination source (e.g. a blue or violet light source) and a sensor for recording light-induced fluorescence (QLF) images for QLF detection of plaque. Red fluorescent regions are indicative of at least plaque and tartar, and the invention provides additional image analysis to distinguish between plaque and tartar.

It is noted that the QLF imaging may also enable detection of a stained spot, carious lesion or crack. Thus, while the system of this disclosure is of primary interest for discriminating between plaque and tartar, it also can, in some implementations, provide detection of a stained spot, carious lesion or crack.

The additional image analysis comprises an analysis of the shape and/or intensity distribution of the regions in which red fluorescence is detected, including external shape characteristics (such as edge sharpness) and internal shape characteristics such as an intensity pattern over area.

The toothbrush has an output system 18 with the primary purpose of providing a notification when plaque is determined to be present. The output system may be a speaker and/or light output. Additionally, or alternatively, it may be a wireless communication system for controlling a remote device such as a smartphone to generate an output message to the user. The output system may also provide brushing guidance to a user. For example, it may adapt existing brushing guidance being provided by a guidance program by increasing the remaining time to brush at the current location when plaque is detected.

In addition to plaque detection, the presence of tartar or other relevant features may also be communicated to the user, or brushing guidance or a brush operation mode may also be changed. For example, at a certain level of tartar users may get a recommendation to visit their dental professional.

This invention is based on the recognition that the red fluorescent image generated by tartar is slightly different to that generated by plaque. The plaque image has a spotty appearance, whereas the tartar image is more homogeneous in intensity and also has straighter edges. This invention makes use of algorithms running on a processor to detect one or more of these differences.

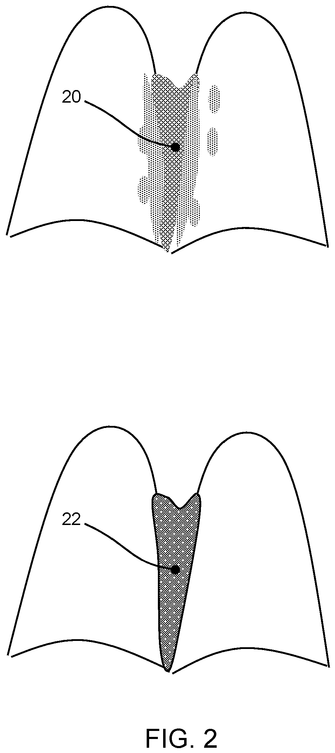

Fig. 2 illustrates schematically a red fluorescence image for plaque (top image) and tartar (bottom image). The top image shows a plaque area 20 and the bottom image show a tartar area 22, at the interdental space.

The plaque image is spottier and rougher at the edges.

In one example, an algorithm implementing a traditional image analysis tool may be employed. For example, the image could be thresholded in the red channel to identify the red fluorescent spot area and circumference. With the rough edge of plaque, the circumference over area ratio will be typically larger. Another method could be to detect the spottiness of the plaque from a simple histogram of the intensity in the red fluorescent area, where the histogram of plaque would give a broader range than that of tartar.

Alternatively, texture features may be extracted, such as local entropy estimation on the individual color intensity channels, and/or local entropy estimation on a combination of channels such as the red channel divided by the green channel. Any other method to estimate texture properties can also be used. A measure of local entropy gives information about the localized fluctuation of intensity and hence discriminates between local areas of uniform intensity and local areas where there are large intensity fluctuations (such as at the edge of spots).

In another example, artificial intelligent (AI) algorithms could be used, e.g., by training a neural network with plaque and tartar images to classify these images correctly. AI has the disadvantage over conventional image analysis that the training requires significant pre-work but may enable detection of features that are not detected by conventional image analysis.

After plaque and tartar fluorescent spots are distinguished, this information can be used in many ways, e.g., the user may obtain feedback on the detected spots in an app or on their brush handle. The brush handle may also give different guidance how to address the different spots in the optimal way and the brush may even change its operation mode based on whether it is on a plaque or tartar position. Tartar spot size may also be followed in time and at a certain level advice may be given to seek dental practitioner treatment. This information may additionally be shared with the dental practitioner so they can decide to call in the user for treatment.

The identification of plaque or tartar from analysis of the QLF image avoids the need for additional imaging, e.g., color and texture analysis of white light images, to make the discrimination. This gives a lower data flow requirement and lower calculation power requirement.

Image analysis options will now be described in greater detail.

The approach of classical (non-trained, non-intelligent) image processing is preferred. The red fluorescent area of interest can be identified using red channel thresholding, and within the identified red fluorescent spot a histogram can be made of the red channel intensity. Alternatively, or additionally, thresholding can be applied to the green channel, or the red channel divided by the green channel, or a difference between the channels.

The red fluorescent spot will have a non-uniform intensity. By setting an intensity threshold, a boundary of the general area of interest may be defined as one plaque or tartar region. One such region may include multiple smaller spots.

After thresholding is applied to define an area of interest, there may be separate areas (e.g., small islands around a central main area of plaque) if there are low red fluorescent corridors below the threshold in the general area of interest. If there are small islands at the edge of the main plaque area, it may be difficult to classify those islands, because a single small round red fluorescent plaque colony could by itself have tartar shape and intensity distribution characteristics (homogeneous with smooth edges). Therefore, it is preferable to define the main area as well as any separate islands as a single area of interest. Such separate islands then increase the chance that the general area is classified as plaque, as such islands will strongly contribute to the inhomogeneity of the area of interest and will drive the classification towards plaque.

Tartar will not likely have such smaller islands, as their boundary is typically sharper. To include such red spots within the area of interest, a minimum distance condition may be applied before identifying two neighboring red fluorescent spots in an image as being separate areas of interest. For example, only if the distance is more than 2mm is a separate area of interest defined.

Other known image processing techniques can be used for defining the separate areas of interest. For example, so-called 'region growing' may be applied, whereby a 'seed' is defined, i.e., a small spot where red fluorescence is detected. Neighboring pixels are then assessed relative to the red fluorescence threshold and using a maximum distance threshold from the seed. The seed grows iteratively and stops growing when the area of interest is complete.

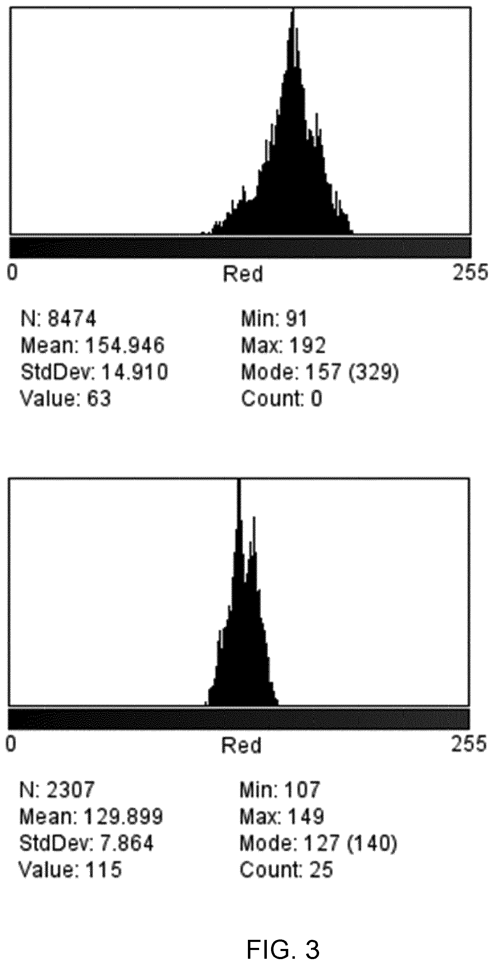

Fig. 3 shows red channel intensity histograms for areas of interest comprising plaque (top image) and tartar (bottom image). The number of pixels (y-axis) within the area of interest is plotted for each red intensity level (x-axis).

The red intensity histogram has a standard deviation (SD) (of the pixel intensity value, which in this example has a range of 256 levels) of 14.9 for the plaque area but is narrower with a SD of 7.9 for the tartar area. Analyzing different plaque and tartar spots, it has been found that a suitable discriminator is that tartar spots have SD < 10 and plaque spots have SD >10. Of course, this is just one example, to show that standard deviation of pixel intensities is one measure which can discriminate between tartar and plaque.

An alternative method may be based on an assessment of texture, for example by calculating local entropy values across the area of interest of the tooth. For example, local entropy values of the red channel or the green channel or the red/green channel may be calculated.

Local entropy is for example calculated on small sub-regions, defined as disks or rectangles, around each pixel. As a result, an entropy map is derived over such elementary regions (rather than a global value). In this way, a measure of the distribution of intensity levels is obtained.

This is one way to obtain a measure of texture. Of course, other ways to measure texture are available such as so-called "histograms of gradients", HoG's. Similarly to local entropy, HoG values may be calculated over an element area (disk or rectangle around a pixel in image) to obtain a map of HoGs and hence obtain information about the distribution of intensity values.

Another option is to apply a spatial Fourier transform to the area of interest. The transform can be applied to the red channel, or to red channel/green channel, etc.
Less homogeneous spots, i.e., plaque, will show relatively more high frequency content in the spatial frequency domain than more homogeneous spots, i.e., tartar.

More generally, any suitable measure of spatial homogeneity of the fluorescence within the area of interest may be obtained.

The rough edge of a plaque area compared to the tartar edge on the side of the teeth may also be detected.

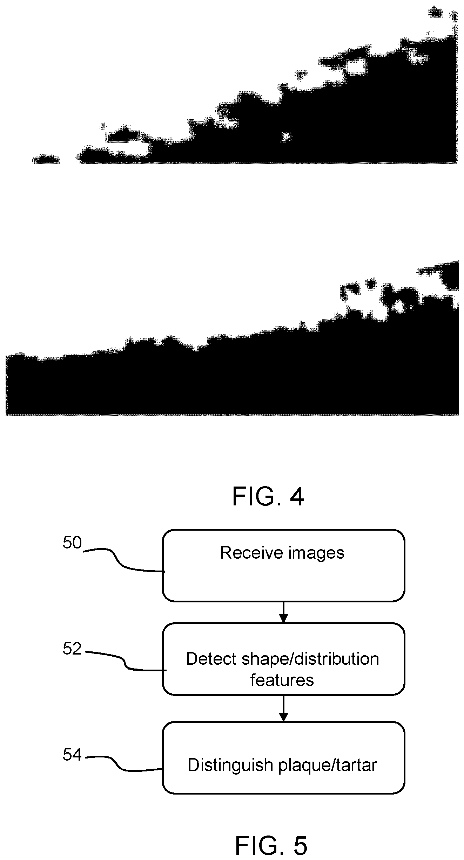

Fig. 4 shows the edges of the red fluorescence images after thresholding the red channel image. The top image shows the plaque area boundary and the bottom image shows the tartar area boundary. Due to the roughness, the length of the border line will be significantly larger with plaque than with tartar.

The examples above use the red channel, but also the green channel intensity and roughness variations can be used. Combinations of red and green channels may further enhance contrast, e.g., red divided by green or green subtracted from red.

Furthermore, the invention is not limited to the generation of red and green channels only. The detected light may be split into more components, for example using a hyperspectral camera. In such a case, different channels or combinations of channels may be processed.

Instead of conventional image processing, a set of training images may be used to train an AI algorithm to recognize tartar and plaque. The algorithm will detect similar features as discussed above, e.g., the patchiness of plaque compared to tartar, but trained AI may also find additional less clearly visible differentiators.

Fig. 5 shows a method for detecting dental plaque.

Intra-oral images are received in step 50, comprising light induced fluorescence images.

Shape and/or intensity distribution features of fluorescent regions in the received images are detected in step 52, and these features are used to discriminate between regions of tartar and regions of plaque in step 54.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A system for detecting dental plaque, comprising:
a camera system (14) for generating intra-oral images comprising a light source and a detector for generating light induced fluorescence images;
a processor (16) for processing the generated images, configured to:
detect shape and/or intensity distribution features of fluorescent regions in the generated images; and
discriminate between regions of tartar and regions of plaque from the shape and/or intensity distribution features.

2. The system of claim 1, wherein the shape features comprise an assessment of spatial homogeneity of fluorescence within the fluorescent regions.

3. The system of claim 1 or 2, wherein the shape and/or intensity distribution features comprise an assessment of edge sharpness.

4. The system of any one of claims 1 to 3, wherein the processor is configured to run a non-intelligent algorithm to analyze the fluorescent regions.

5. The system of claim 4, wherein the processor is configured to determine a circumference to area ratio to determine an edge sharpness.

6. The system of claim 4 or 5, wherein the processor is configured to perform red or green channel thresholding, generate histogram data, and analyze the histogram data to discriminate between regions of tartar and regions of plaque.

7. The system of claim 4 or 5, wherein the processor is configured to determine a spatial homogeneity of fluorescence by:
performing measures of local entropy of the fluorescent regions; or
performing spatial frequency analysis of the fluorescent regions.

8. The system of any one of claims 1 to 3, wherein the processor is configured to run a trained AI algorithm to analyze the fluorescent regions.

9. The system of any one of claims 1 to 8, further comprising an output system (18) configured to provide a notification when plaque is determined to be present.

10. The system of claim 9, wherein the output system is further configured to provide brushing guidance to a user.

11. An oral care device (10) comprising:
an oral care element (12); and
the system (14,16,18) of any one of claims 1 to 10.

12. The oral care device of claim 11 comprising a powered toothbrush, wherein the oral care element comprises a driven toothbrush head, wherein the processor is configured to adapt operating parameters of the powered toothbrush in response to the detection of regions of plaque.

13. A method for detecting dental plaque, comprising:
receiving intra-oral images comprising light induced fluorescence images;
detecting shape and/or intensity distribution features of fluorescent regions in the received images; and
discriminating between regions of tartar and regions of plaque from the shape and/or intensity distribution features.

14. The method of claim 13, wherein the shape and/or intensity distribution features comprise an assessment of spatial homogeneity of fluorescence within the fluorescent regions and/or an assessment of edge sharpness.

15. A computer program comprising computer program code which is adapted, when said program is run on a processor, to perform the method of any one of claims 13 to 14.
